# EUROPÄISCHE PATENTSCHRIFT

(11) **EP 0 984 664 B1**
(45) Veröffentlichungstag und Bekanntmachung des Hinweises auf die Patenterteilung: **02.01.2008**
(21) Anmeldenummer: 98121496.8
(22) Anmeldetag: 12.11.1998
(51) Int. Cl.: H04R 25/00, A61F 11/04, A61N 1/378, A61F 2/02

(54) **Implantierbare Vorrichtung mit einer eine Empfangsspule aufweisenden Ladestromeinspeiseanordnung**
Implantable device with a current charging arrangement containing a receiver coil
Appareil implantable avec un dispositif du courant de charge comportant une bobine de réception

(30) Priorität: 20.08.1998 DE 19837913
(43) Veröffentlichungstag der Anmeldung: 08.03.2000
(73) Patentinhaber: Cochlear Limited, Lane Cove, NSW 2066 (AU)
(72) Erfinder: Leysieffer, Hans Dr.-Ing., 82024 Taufkirchen (DE)
(74) Vertreter: Schwan, Gerhard

(56) Entgegenhaltungen:
- EP-A- 0 291 325
- WO-A-94/08539
- DE-C- 19 645 371
- US-A- 4 495 917
- US-A- 5 272 283

## Beschreibung

Die Erfindung betrifft eine implantierbare Vorrichtung mit einer mindestens eine Empfangsspule aufweisenden oder aus einer solchen Spule bestehenden Ladestromeinspeiseanordnung, in die über eine extrakorporal angeordnete Ladevorrichtung Energie transkutan elektromagnetisch einspeisbar ist, einer über die Ladestromeinspeiseanordnung mehrfach nachladbaren elektrochemischen Batterie, und einem von der Batterie mit elektrischer Energie versorgten Hauptmodul, wobei die Komponenten der implantierbaren Vorrichtung mit Ausnahme der Empfangsspule in einer Gehäuseanordnung aus biokompatiblem Metall untergebracht sind und wobei die Empfangsspule außerhalb der Gehäuseanordnung sitzt und mit dem übrigen Teil der implantierbaren Vorrichtung elektrisch gekoppelt ist.

Aus der US-PS 5,279,292 sind eine Hörhilfe oder ein Tinnitus-Maskierer bekannt, bei denen ein Hauptmodul und ein Energieversorgungsmodul mit nachladbarer elektrochemischer Batterie in einem gemeinsamen Gehäuse oder jeweils in einem separaten Gehäuse untergebracht sein können, wobei das gemeinsame Gehäuse oder das Gehäuse des Energieversorgungsmoduls auch die Empfangsspule aufnimmt, in die über die extrakorporal angeordnete Ladevorrichtung Energie transkutan elektromagnetisch einspeisbar ist. Solche Implantatgehäuse müssen hermetisch gasdicht sein. Daher hat man für das die Empfangsspule enthaltende Gehäuse in Anbetracht der transkutanen Ladestromeinspeisung keramische Werkstoffe, zum Beispiel Al₂O₃ oder dergleichen, vorgesehen. Keramikgehäuse sind aber kostspielig und relativ stoßempfindlich. Die US-PS 5,279,292 beschreibt auch eine implantierbare Vorrichtung mit einer Ladestromeinspeiseanordnung, bei der Energie perkutan in eine außerhalb des Implantatgehäuses befindliche Empfangsspule eingespeist wird, zu der ein durch die Haut hindurchragender Ferritkern gehört. Jede perkutane Anordnung stellt jedoch eine potentielle Infektions- und Verletzungsquelle dar. Das Vorhandensein eines Implantats wird von außen störend sichtbar. Durch den Ferritkern sind kernspintomografische Untersuchungen des Implantatträgers und ähnliches in unerwünschter Weise ausgeschlossen.

Aus der US-PS 5,702,431 ist ferner ein transkutanes Wiederaufladesystem für batteriegespeiste implantierbare medizinische Geräte, insbesondere Defibrillatoren, bekannt, bei dem eine Empfangsspule einer Ladestromeinspeiseanordnung außerhalb des Gerätegehäuses angeordnet und mit dem implantierbaren Gerät elektrisch gekoppelt ist und bei dem das implantierbare Gerät in einem Gehäuse aus Titan oder rostfreiem Stahl untergebracht ist.

Der Erfindung liegt die Aufgabe zugrunde, eine implantierbare Vorrichtung zu schaffen, die ohne Keramikgehäuse auskommt, aber gleichwohl für den Implanteur einfach zu handhaben ist.

Diese Aufgabe wird bei einer implantierbaren Vorrichtung mit den Merkmalen des Oberbegriffs des Anspruchs 1 erfindungsgemäß dadurch gelöst, daß die Empfangsspule aus mindestens einem mit einem elektrisch isolierenden Material umhüllten metallischen Leiter gewickelt, von einem biokompatiblen Polymer umgeben und mit mindestens einem Teil der Gehäuseanordnung zusätzlich mechanisch verbunden ist und somit eine bauliche Einheit mit dem mindestens einem Teil bildet.

Im Falle der implantierbaren Vorrichtung nach der Erfindung bildet die Empfangsspule der Ladestromeinspeiseanordnung mit mindestens einem Teil der Gehäuseanordnung der Vorrichtung eine bauliche Einheit, die beim Implantationsvorgang bequem und übersichtlich als Ganzes zu handhaben ist. Zugleich lassen sich sämtliche Anforderungen sowohl an die Biokampatibilität der Vorrichtung als auch an die Eignung zur transkutanen Übertragung von elektromagnetischer Energie für das Laden der Batterie erfüllen, ohne daß auf kostspielige und mechanisch empfindliche Keramikgehäuse zurückgegriffen werden muß.

Bei der implantierbaren Vorrichtung kann es sich grundsätzlich um eine beliebige implantierbare medizinische oder biologische Vorrichtung handeln, so unter anderem um ein aktives, elektronisches Hörimplantat, einen Herzschrittmacher, einen Defibrillator, einen Drogenspender, einen Nerven- oder Knochenwachstumsstimulator, einen Neuro- oder Retinastimulator, ein Schmerzunterdrückungsgerät oder dergleichen.

Vorteilhafte Weiterbildungen der Erfindung sind Gegenstand der Unteransprüche. Bevorzugt wird für eine hermetisch dichte Ausbildung der metallischen Gehäuseanordnung gesorgt. Unter hermetischer Dichtheit wird vorliegend vorzugsweise hermetische Gasdichtheit nach Mil-Std 883 D verstanden. Durch diese Ausführung wird gewährleistet, daß weder flüssige noch gasförmige Substanzen aus der implantierten Gehäuseanordnung austreten oder in diese eindringen können.

Die Empfangsspule kann je nach den erforderlichen Abmessungen der Vorrichtungskomponenten und den räumlichen Gegebenheiten im Bereich der Implantationsstelle an einer Außenseite eines mindestens das Hauptmodul oder mindestens die Batterie aufnehmenden Metallgehäuses festgelegt sein.

Das biokompatible Polymer, vorzugsweise Silikon, Polyurethan, Polyimid, Polytetrafluorethylen (PTFE), Polymethan, Parylen oder dergleichen, kann zum einen zur Erhöhung des mechanischen Zusammenhalts der Spule selbst, zum anderen zur mechanischen Anbindung der Spule am entsprechenden Gehäuse dienen.

Eine besonders kompakte Bauform kann erhalten werden, wenn die Empfangsspule auf mindestens einen Teil einer Breitseite des Metallgehäuses des Hauptmoduls bzw. des metallischen Außen- oder Schutzgehäuses des Batteriemoduls aufgesetzt ist, wobei zweckmäßig die Spulenachse der Empfangsspule mindestens näherungsweise senkrecht zu dieser Breitseite steht.

Dagegen ist es für eine besonders effektive transkutane Energieübertragung günstig, wenn die Empfangsspule seitlich angrenzend an mindestens einen Teil einer Schmalseite des Metallgehäuses des Hauptmoduls bzw. des metallischen Außen- oder Schutzgehäuses des Batteriemoduls angeordnet ist, wobei vorteilhaft die Spulenachse der Empfangsspule mindestens näherungsweise parallel zu dieser Schmalseite steht.

Die Empfangsspule kann an dem Metallgehäuse des Haupt- oder Batteriemoduls flexibel festgelegt sein, wodurch erreicht wird, daß die Einheit aus Spule und Gehäuse sich dem Implantationsort geometrisch besser anpassen kann. Dabei besteht die flexible Verbindung zwischen der Empfangsspule und dem betreffenden Gehäuse zweckmäßig aus biokompatiblem Polymer, für das insbesondere die vorstehend im Zusammenhang mit der Umhüllung der Empfangsspule genannten Polymere in Betracht kommen.

Bei dem Leiterwerkstoff der Empfangsspule handelt es sich bevorzugt um einen biokompatiblen metallischen Werkstoff, insbesondere ein Metall, das aus der aus Gold, Platin, Niob, Tantal, Iridium und deren Legierungen bestehenden Gruppe ausgewählt ist. Gold, vor allem reines Gold, ist wegen der Kombination von hoher elektrischer Leitfähigkeit und hervorragender Biokompatibilität besonders geeignet. Grundsätzlich kann als Leiterwerkstoff für die Empfangsspule aber auch ein als solches nicht biokompatibles Metall, zum Beispiel Kupfer, vorgesehen sein, wenn die Biokompatibilität der Gesamtanordnung auf andere Weise sichergestellt ist, beispielsweise durch das Hüllmaterial des Empfangsspulenleiters und/oder durch das die Empfangsspule umgebende Polymer.

Bei Ausbildung der Empfangsspule als kernlose Spule (Luftspule) kann die implantierbare Vorrichtung insgesamt frei von ferromagnetischen Werkstoffen gehalten werden. Dadurch ist der Implantatträger keinen Beschränkungen beispielsweise hinsichtlich einer Kernspintomografie oder dergleichen ausgesetzt.

In weiterer Ausgstaltung der Erfindung kann die Empfangsspule gegenüber dem mit ihr mechanisch verbundenen Metallgehäuse um einen Winkel im Bereich von 5° bis 25° abgeknickt sein. Dadurch ergibt sich eine Einheit aus Spule und entsprechendem Gehäuse, die sich besonders zur Implantation an der Außenseite der Schädelkalotte des Menschen, insbesondere im Bereich des Planum Mastoideum eignet, wie dies z.B. bei mindestens teilweise implantierbaren Hörhilfen, Tinnitus-Maskiereren oder Retinastimulatoren der Fall ist und bereits in der DE-Patentanmeldung 198 29 637.1 beschrieben wurde.

Es versteht sich, daß die Batterie auch ein oder mehr an das Hauptmodul anschließbare(s) Nebenmodul(e) mit elektrischer Energie versorgen kann, bei denen es sich um aktorische und/oder sensorische Komponenten handeln kann.

Nachfolgend sind vorteilhafte Ausführungsformen der Erfindung anhand der Zeichnungen näher erläutert. Es zeigt:
- Fig. 1: eine schematische Darstellung im Schnitt durch eine implantierbare Vorrichtung mit Haupt- und Nebenmodulen, wobei die Spule einer Ladestromeinspeiseanordnung dem Hauptmodul zugeordnet und in einer Polymerumhüllung untergebracht ist;
- Fig. 2: eine Schnittdarstellung entlang der Linie II-II des Hauptmoduls gemäß Fig. 1;
- Fig. 3: eine Schnittdarstellung ähnlich Fig. 2 für eine abgewandelte Ausführungsform des Hauptmoduls;
- Fig. 4: eine Draufsicht auf ein Hauptmodul mit abgewandelter Anordnung der Spule der Ladestromeinspeiseanordnung;
- Fig. 5: eine schematische Schnittdarstellung eines Hauptmoduls mit zugeordneter Empfangsspule entsprechend einer weiter abgewandelten Ausführungsform;
- Fig. 6: eine schematische Schnittdarstellung ähnlich Fig. 5, jedoch mit abgeknicktem Hauptmodulgehäuse;
- Fig. 7: eine schematische Darstellung im Schnitt durch eine weitere implantierbare Vorrichtung mit an das Hauptmodul flexibel angekoppeltem Batteriemodul, wobei die Spule der Ladestromeinspeiseanordnung dem Hauptmodul zugeordnet ist;
- Fig. 8: eine schematische Darstellung eines Hauptmoduls einer implantierbaren Vorrichtung mit daran angebrachter Spule sowie eines an das Hauptmodul flexibel angekoppelten Batteriemoduls;
- Fig. 9: eine Ausführungsform ähnlich Fig. 8, bei welcher die Empfangsspule jedoch mit dem Schutzgehäuse der Batterie mechanisch verbunden ist; sowie
- Fig. 10: einen Querschnitt eines isolierten Leiters der Empfangsspule.

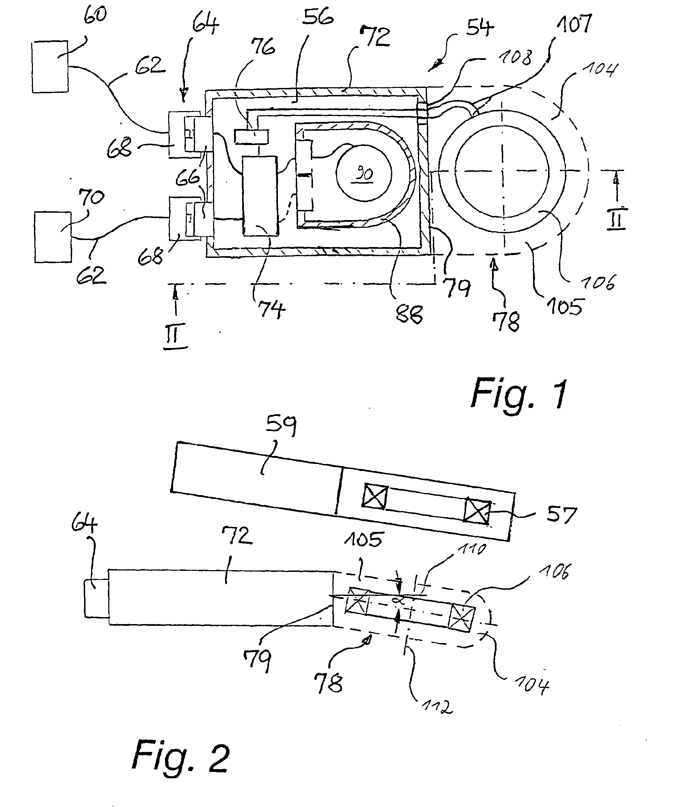

Eine in Fig. 1 insgesamt mit 54 bezeichnete implantierbare Vorrichtung umfaßt ein Hauptmodul 56, sowie als Nebenmodule eine sensorische und eine aktorische Komponente 60 bzw. 70. Die Nebenmodule 60 und 70 sind jeweils über eine flexible Anschlußleitung 62 und ein als Ganzes mit 64 bezeichnetes Koppelorgan mit dem Hauptmodul verbunden. Das sensorische Nebenmodul kann im Falle einer implantierbaren Hörhilfe ein Mikrofon sein, während es sich bei dem aktorischen Nebenmodul um einen an die Gehörknöchelchenkette mechanisch oder an die flüssigkeitsgefüllten Räume des Innenohres hydromechnisch ankoppelbaren elektromechanischen Wandler handeln kann, wie dies in der US-PS 5,277,694, der US-PS 5,411,467 und der EP-A-0 831 674 im einzelnen erläutert ist. Das Koppelorgan 64 besitzt eine dem Hauptmodul 56 zugeordnete erste Hälfte 66 sowie eine mit der ersten Hälfte 66 lösbar gekoppelte nebenmodulseitige zweite Hälfte 68, in welche die flexible Anschlußleitung 62 mündet. Das Koppelorgan 64 kann insbesondere in der aus der US-PS 5,755,743 bekannten Weise aufgebaut sein, um auf geringem Raum für eine vorzugsweise hermetisch dichte gegenseitige Verbindung zu sorgen. Es versteht sich, daß sämtliche in den Figuren durch eine einfache Linie vereinfacht wiedergegebenen Leitungen je nach den Komponenten, die sie verbinden, prinzipiell ein- oder mehrpolig ausgeführt sein können. Entsprechendes gilt für Koppelorgane und Leitungsdurchführungen durch Gehäuse oder Gehäuseteile.

Ein Hauptmodulgehäuse 72 des Hauptmoduls 56 nimmt eine Signalverabeitungselektronik 74, eine Lade-/Entladeelektronik 76 und eine nachladbare elektrochemische Batterie 90 samt zugehörigem Schutzgehäuse 88 hermetisch dicht auf und ist aus einem biokompatiblen Metall gefertigt. Die Signalverarbeitungselektronik 74 steuert die aktorische Komponente 70 nach einem gespeicherten Programm in Abhängigkeit von den Signalen der sensorischen Komponente 60 und ist an die beiden Komponenten über die Koppelorgane 64 angeschlossen, deren erste Hälften 66 im Hauptmodulgehäuse 72 hermetisch dicht integriert sind. Die Lade-/Entladeelektronik 76 bildet einen Knotenpunkt zwischen der Signalverarbeitungselektronik 74, der Batterie 90 und einer Ladestromeinspeiseanordnung 78, und sie dient der Energieverteilung zwischen diesen Komponenten. Zu der Ladestromeinspeiseanordnung 78 gehört insbesondere eine Empfangsspule 106, die zum Aufladen der Batterie 90 mit einer außerhalb des Körpers angeordneten Sendespule 57 einer Ladevorrichtung 59 induktiv gekoppelt werden kann, wie dies beispielsweise aus der US-PS 5,279,292 bekannt ist. Die Ladestromeinspeiseanordnung 78 kann gegebenenfalls nicht gezeigte weitere Komponenten enthalten, z.B. einen Kondensator zum Aufbau eines Schwingkreises. Die Empfangsspule 106 ist aus einem mit elektrisch isolierendem und vorzugsweise biokompatiblem Material 111 umhülltem metallischem Leiter 109 (Fig. 10) mit möglichst niedrigem spezifischem elektrischem Widerstand oder aus mehreren solchen Leitern gewickelt und mit einem biokompatiblen Polymer 104, beispielsweise einem Silikon, umgossen.

Die aus der Polymerumhüllung 104 und der Empfangsspule 106 bestehende Einheit 105 ist bei der Ausführungsform gemäß den Figuren 1 und 2 mit der von dem Koppelorgan 64 abgewendeten Schmalseite 79 des metallischen Hauptmodulgehäuses 72 mechanisch fest verbunden. Beispielsweise kann die Einheit 105 mit dem Hauptmodulgehäuse 72 verklebt sein. Die Polymerumhüllung 104 kann aber auch an das Hauptmodulgehäuse 72 angeformt oder angespritzt sein. Die Spule 106 ist mit der Lade-/Entladeelektronik 76 über Anschlüsse 107 elektrisch verbunden, die über eine hermetisch dichte Durchführung 108 aus dem Hauptmodulgehäuse 72 herausgeführt sind.

Zum Laden wird, wie in Fig. 2 angedeutet ist, die Sendespule 57 der extrakorporalen Ladevorrichtung 59 mit der Empfangsspule 106 ausgerichtet, und elektromagnetische Energie wird transkutan von der Sendespule zur Empfangsspule übertragen.

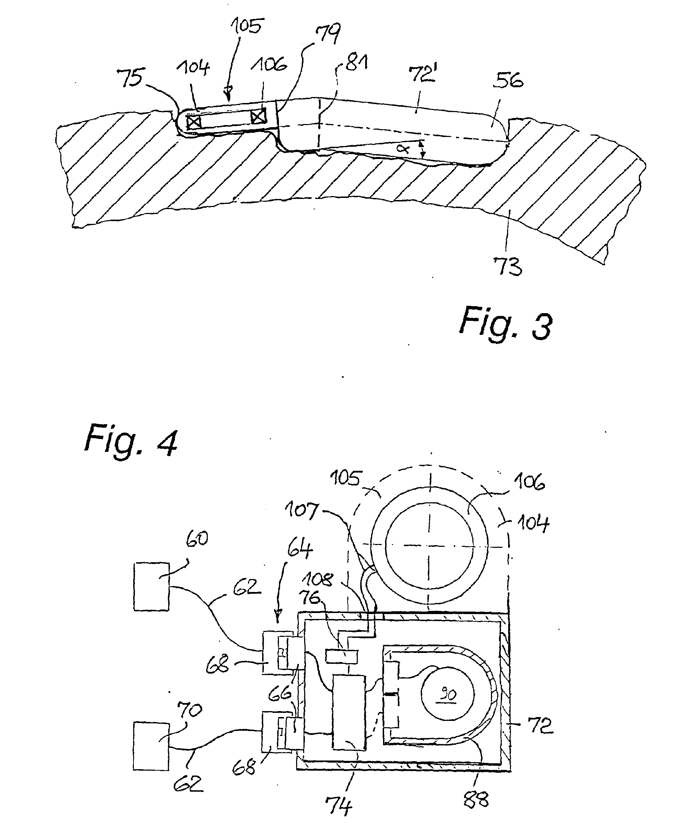

Die Schmalseite 79 des Hauptmodulgehäuses 72 liegt senkrecht zu einer in Richtung der größten Ausdehnung des Gehäuses 72 verlaufenden Geraden 110. Eine senkrecht zur Achse 112 der Spule 106 verlaufende Gerade bildet mit der Geraden 110 einen Winkel α im Bereich von 5° bis 25°, vorzugsweise im Bereich von 7° bis 15°. Ein solches Abknicken der aus dem Hauptmodulgehäuse 72 und der Einheit 105 bestehenden Anordnung erlaubt es, bei einer Implantation der Vorrichtung in einem an der Außenseite der Schädelkalotte 73 (Fig. 3), insbesondere im Bereich des Planum Mastoideum, ausgebildeten Knochenbettes 75 bei vorgegebener Größe, insbesondere Tiefe, des Knochenbettes ein vergleichsweise großes Gehäusevolumen bereitzustellen und gleichzeitig ein Überstehen des Gehäuses über den Außenrand des Knochenbettes ganz oder mindestens weitgehend zu vermeiden, wie dies im einzelnen in der früheren DE-Patentanmeldung 198 29 637.1 erläutert ist.

Während bei dem Ausführungsbeispiel der Figuren 1 und 2 die Knickstelle der Vorrichtung 54 mit der Gehäuseschmalseite 79 zusammenfällt, zeigt Fig. 3 eine Ausführungsform, bei welcher eine entsprechende Knickstelle 81 im Bereich der Längserstreckung des Gehäuses 72' des Hauptmoduls liegt.

Die aus der Polymerumhüllung 104 und der Empfangsspule 106 bestehende Einheit 105 kann an dem Hauptmodulgehäuse 72 oder 72' auch an anderer Stelle als an der Schmalseite 79 angebracht sein. So zeigt die Drausicht gemäß Fig. 4 ein Ausführungsbeispiel, bei dem die Einheit 105 an die eine Breitseite 83 des Gehäuses 72 angesetzt ist.

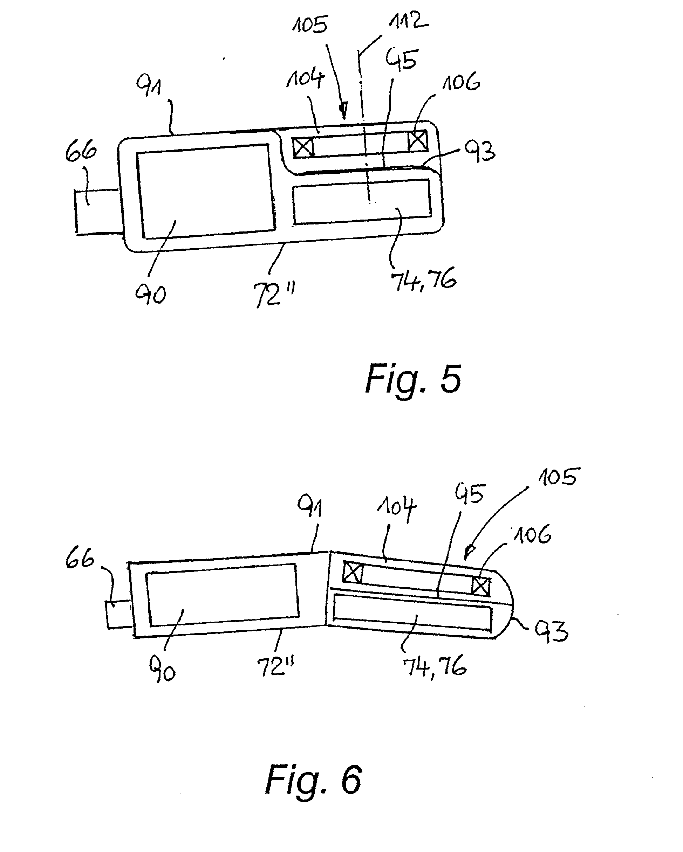

In der Praxis erfordert in der Regel die aufladbare Batterie 90 zusammen mit einem der Batterie gegebenenfalls zugeordneten Schutzsystem eine Höhenabmessung, die größer ist als die notwendige Höhenabmessung der Elektronikbaugruppen 74 und 76 der Vorrichtung. Kommt es bei der Implantation darauf an, daß das Implantat hinsichtlich seiner beiden anderen Abmessungen, das heißt der Breiten- und Längenabmessung, besonders kompakt ist, kann die Anordnung vorteilhaft in der in den Figuren 5 und 6 veranschaulichten Weise getroffen sein. Dabei weist das Hauptmodulgehäuse 72" einen zur Aufnahme der Batterie 90 bestimmten höheren Gehäuseabschnitt 91 und einen die Elektronikbaugruppen 74 und 76 aufnehmenden Gehäuseabschnitt 93 mit gegenüber dem Gehäuseabschnitt 91 verminderter Höhe auf Im Bereich des durch die Gehäuseabstufung entstehenden Raumes ist die Empfangsspule 106 zusammen mit ihrer Polymerummantelung 104 auf der Breitseite 95 des niedrigeren Gehäuseabschnittes 93 angebracht, wobei die Spulenachse 112 im wesentlichen senkrecht zu der Breitseite 95 steht. Wie dargestellt können dabei die aus Empfangsspule 106 und Polymerummantelung 104 bestehende Einheit 105 und der Gehäuseabschnitt 93 zusammen mindestens näherungsweise die gleiche Höhe wie der Gehäuseabschnitt 91 haben. Zu berücksichtigen ist allerdings, daß eine Anordnung der Einheit 105 auf statt neben dem Metallgehäuse 72'' zur Folge hat, daß beim Laden die von der Sendespule erzeugte elektromagnetische Durchflutung nicht nur die Empfangsspule 106, sondern auch metallische Gehäuseteile durchsetzen muß. Um dabei übermäßige Energieverluste durch in den metallischen Gehäuseteilen induzierte Wirbelströme und damit eine unzulässige Aufheizung des Metallgehäuses zu vermeiden, sind geeignete Gegenmaßnahmen zu treffen, wie zum Beispiel Wahl einer verhältnimäßig niedrigen Frequenz des von der Sendespule erzeugten Wechselfeldes und/oder Minimierung der Wandstärke der von diesem Feld beim Laden durchsetzten Gehäusewandteile.

Entsprechend Fig. 6 kann, falls erwünscht, der Gehäuseabschnitt 93 samt Einheit 105 gegenüber dem Gehäuseabschnitt 91 in ähnlicher Weise abgeknickt sein, wie dies vorstehend anhand der Figuren 1 und 2 für das Abknicken der Einheit 105 gegenüber dem Gehäuse 72 erläutert ist.

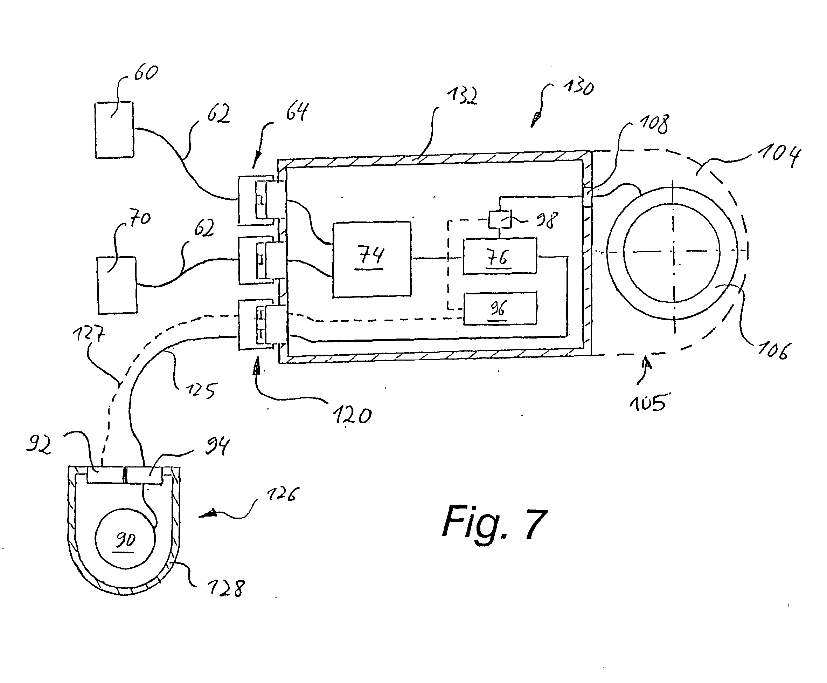

Bei der in Fig. 7 dargestellten Ausführungsform nimmt ein Hauptmodulgehäuse 132 als Hauptmodul 130 neben der Signalverarbeitungselektronik 74 und der Lade-/Entladeelektronik 76 eine Auswerteelektronik 96 und ein Schaltorgan 98 auf An dem Hauptmodulgehäuse 132 ist die aus Empfangsspule 106 und Polymerummantelung 104 bestehende Einheit 105 angebracht. Die Batterie 90 ist in diesem Fall in einem von dem Hauptmodulgehäuse 132 getrennten, hermetisch dichten Schutzgehäuse 128 eines Batteriemoduls 126 untergebracht. Das Schutzgehäuse 128 ist ebenso wie das Hauptmodulgehäuse 132 aus biokompatiblem Metall gefertigt. Das Batteriemodul 126 ist über eine Energieleitung 125 sowie ein insgesamt mit 120 bezeichnetes Koppelorgan mit der Lade-/Entladeelektronik 76 lösbar verbunden. Zwischen der Batterie 90 und der Energieleitung 125 liegt bei diesem Ausführungsbeispiel ein als Öffner ausgebildetes Schaltorgan 94, welches an dem Schutzgehäuse 128 festgelegt ist und von einem Detektororgan 92, beispielsweise einer wölbbaren Membran in einer Außen- oder Trennwand des Schutzgehäuses 128, mechanisch betätigt wird, wenn dem Detektororgan 92 bei einem unzulässigen Betriebszustand der Batterie 90 eine Formänderung aufgeprägt wird. Das Detektororgan 92 steht über eine Signalleitung 127 und das Koppelorgan 120 mit der Auswerteelektronik 96 in Verbindung, die ihrerseits an das Schaltorgan 98 angeschlossen ist. Die Auswerteelektronik 96 überwacht den Zustand des Detektororgans 92 und betätigt in Abhängigkeit davon das als Öffner ausgestaltete Schaltorgan 98, welches im Strompfad zwischen der Empfangsspule 106 und der Lade-/Entladeelektronik 76 plaziert ist. Der Formänderungszustand des Detektororgans 92 wird beispielsweise über einen elektrischen Dehnungsmeßstreifen kontrolliert. Bei Überschreitung einer vorgegebenen Grenzformänderung des Detektororgans 92 unterbricht das Schaltorgan 98 eine weitere Energiezufuhr aus der Einheit 105 unabhängig von der Funktion des Schaltorgans 94, so daß eine Redundanz gegeben ist.

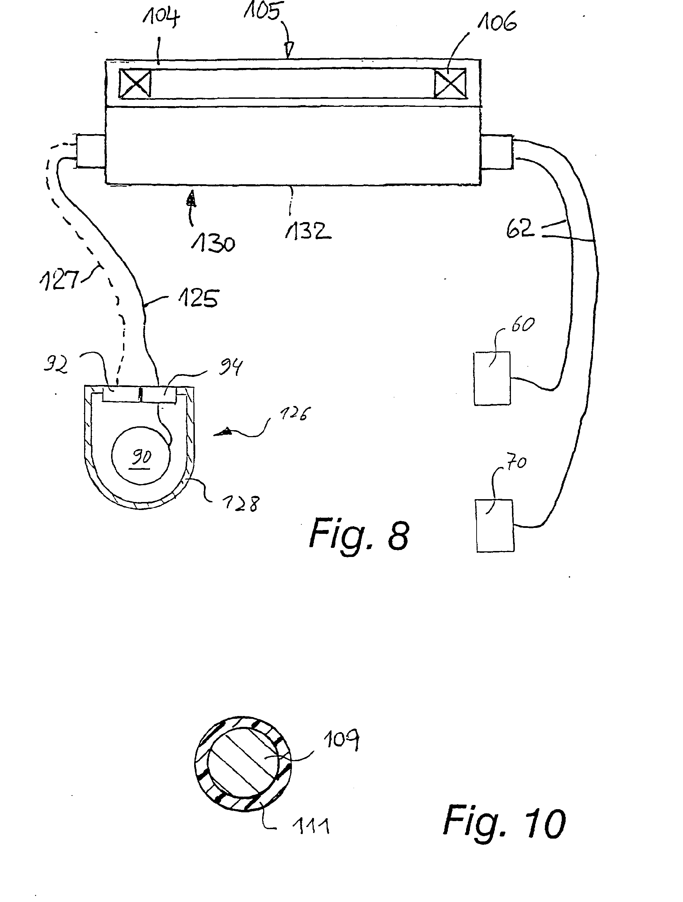

Die Ausführungsform der Fig. 8 unterscheidet sich von derjenigen der Fig. 7 im wesentlichen nur dadurch, daß die Empfangsspuleneinheit 105 nicht seitlich an dem Hauptmodulgehäuse 132 angebracht, sondern auf eine Breitseite des Hauptmodulgehäuses 132 aufgesetzt ist.

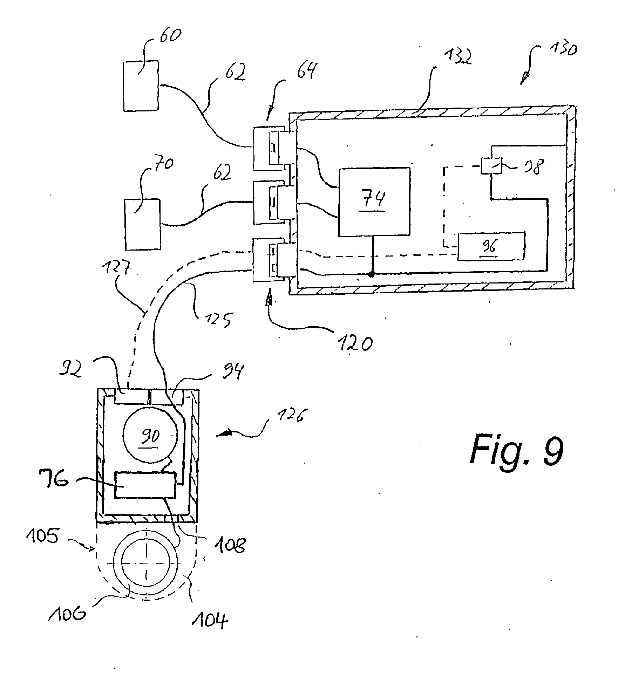

Bei der Ausführungsform gemäß Fig. 9 ist abweichend von den Ausführungsbeispielen der Figuren 7 und 8 die Empfangsspuleneinheit 105 nicht an dem Hauptmodulgehäuse 132, sondern an dem metallischen Schutzgehäuse 128 der Batterie 90 befestigt. Außerdem ist die Lade-/Entladeelektronik 76 Bestandteil des Batteriemoduls 126. Während in Fig. 9 eine seitliche Anbringung der Einheit 105 an dem Schutzgehäuse 128 dargestellt ist, kann die Einheit 105 auch analog der Anordnung nach Fig. 8 auf eine Breitseite des Gehäuses 128 aufgesetzt sein.

Im Rahmen der Erfindung sind zahlreiche weitere Abwandlungen und Ausgestaltungen möglich. Zum Beispiel kann die Empfangsspule fakultativ auch als Sendespule einer vorzugsweise bidirektionalen Telemetrieschaltung genutzt werden, um beispielsweise über die relative Position der Empfangsspule zur Sendespule der Ladevorrichtung und/oder den Ladezustand der Batterie Informationen transkutan auszutauschen. Für solche Zwecke kann gegebenenfalls eine gesonderte Sendespule auch zusätzlich zu der Empfangsspule vorgesehen sein, wobei die Sendespule gleichfalls außerhalb der Gehäuseanordnung sitzt und mit dem übrigen Teil der implantierbaren Vorrichtung elektrisch gekoppelt sowie aus biokompatiblem Metall, insbesondere Gold, gefertigt, von einem biokompatiblen Polymer umgeben und mit mindestens einem Teil der Gehäuseanordnung mechanisch verbunden ist.

## Patentansprüche

1. Implantierbare Vorrichtung mit einer mindestens eine Empfangsspule (106) aufweisenden oder aus einer solchen Spule bestehenden Ladestromeinspeiseanordnung (78), in die über eine extrakorporal angeordnete Ladevorrichtung (59) Energie transkutan elektromagnetisch einspeisbar ist, einer über die Ladestromeinspeiseanordnung mehrfach nachladbaren elektrochemischen Batterie (90), und einem von der Batterie mit elektrischer Energie versorgten Hauptmodul (56, 130), wobei die Komponenten der implantierbaren Vorrichtung mit Ausnahme der Empfangsspule in einer Gehäuseanordnung (72, 72', 72'', 128, 132) aus biokompatiblem Metall untergebracht sind und wobei die Empfangsspule außerhalb der Gehäuseanordnung sitzt und mit dem übrigen Teil der implantierbaren Vorrichtung (54) elektrisch gekoppelt ist, **dadurch gekennzeichnet, daß** die Empfangsspule (106) aus mindestens einem mit einem elektrisch isolierenden Material (111) umhüllten metallischen Leiter (109) gewickelt, von einem biokompatiblen Polymer (104) umgeben und mit mindestens einem Teil der Gehäuseanordnung (72, 72', 72", 128, 132) zusätzlich mechanisch verbunden ist und somit eine bauliche Einheit mit dem mindestens einem Teil bildet.

2. Implantierbare Vorrichtung nach Anspruch 1, **dadurch gekennzeichnet, daß** die metallische Gehäuseanordnung (72, 72', 72'', 128, 132) hermetisch dicht ausgebildet ist.

3. Implantierbare Vorrichtung nach Anspruch 1 oder 2, **dadurch gekennzeichnet, daß** die Empfangsspule (106) an einer Außenseite eines mindestens das Hauptmodul (56, 130) aufnehmenden Metallgehäuses (72, 72', 72'', 132) festgelegt ist.

4. Implantierbare Vorrichtung nach Anspruch 1 oder 2, **dadurch gekennzeichnet, daß** die Empfangsspule (106) an einer Außenseite eines mindestens die Batterie (90) aufnehmenden metallischen Außen- oder Schutzgehäuses (128) festgelegt ist.

5. Implantierbare Vorrichtung nach Anspruch 3 oder 4, **dadurch gekennzeichnet, daß** die Empfangsspule (106) an dem betreffenden Gehäuse (72, 72', 72'', 128, 132) über das sie umgebende Polymer (104) fixiert ist.

6. Implantierbare Vorrichtung nach einem der Ansprüche 3 bis 5, **dadurch gekennzeichnet, daß** die Empfangsspule (106) auf mindestens einen Teil einer Breitseite (95) des Metallgehäuses (72'', 132) des Hauptmoduls (56, 130) bzw. des metallischen Außen- oder Schutzgehäuses (128) des Batteriemoduls (126) aufgesetzt ist.

7. Implantierbare Vorrichtung nach Anspruch 6, **dadurch gekennzeichnet, daß** die Spulenachse (112) der Empfangsspule (106) mindestens näherungsweise senkrecht zu der einen Breitseite (95) des Metallgehäuses (72'', 132) des Hauptmoduls (56, 130) bzw. des metallischen Außen- oder Schutzgehäuses (128) des Batteriemoduls (126) steht.

8. Implantierbare Vorrichtung nach einem der Ansprüche 3 bis 5, **dadurch gekennzeichnet, daß** die Empfangsspule (106) seitlich angrenzend an mindestens einen Teil einer Schmalseite (79) des Metallgehäuses (72, 72', 132) des Hauptmoduls (56, 130) bzw. des metallischen Außen- oder Schutzgehäuses (128) des Batteriemoduls (126) angeordnet ist.

9. Implantierbare Vorrichtung nach Anspruch 8, **dadurch gekennzeichnet, daß** die Spulenachse (112) der Empfangsspule (106) mindestens näherungsweise parallel zu der einen Schmalseite (79) des Metallgehäuses (72, 72', 132) des Hauptmoduls (56, 130) bzw. des metallischen Außen- oder Schutzgehäuses (128) des Batteriemoduls (126) steht.

10. Implantierbare Vorrichtung nach Anspruch 8 oder 9, **dadurch gekennzeichnet daß** die Empfangsspule (106) gegenüber dem mit ihr mechanisch verbundenen Metallgehäuse (72, 72', 132) um einen Winkel im Bereich von 5° bis 25° abgeknickt ist.

11. Implantierbare Vorrichtung nach einem der Ansprüche 8 bis 10, **dadurch gekennzeichnet, daß** die Empfangsspule (106) an dem Metallgehäuses (72, 72', 132) des Hauptmoduls (56, 130) bzw. an dem metallischen Außen- oder Schutzgehäuse (128) des Batteriemoduls (126) flexibel festgelegt ist.

12. Implantierbare Vorrichtung nach Anspruch 11, **dadurch gekennzeichnet, daß** die flexible Verbindung zwischen der Empfangsspule (106) und dem betreffenden Gehäuse (72, 72', 72'', 128, 132) aus biokompatiblem Polymer besteht.

13. Implantierbare Vorrichtung nach einem der vorhergehenden Ansprüche, **dadurch gekennzeichnet, daß** der Leiter (109) der Empfangsspule (106) aus biokompatiblem Metall besteht.

14. Implantierbare Vorrichtung nach einem der vorhergehenden Ansprüche, **dadurch gekennzeichnet, daß** der Leiter (109) der Empfangsspule (106) aus einem Metall gefertigt ist, das aus der aus Gold, Platin, Niob, Tantal, Iridium und deren Legierungen bestehenden Gruppe ausgewählt ist.

15. Implantierbare Vorrichtung nach Anspruche 14, **dadurch gekennzeichnet, daß** der Leiter (109) der Empfangsspule (106) aus reinem Gold gefertigt ist.

16. Implantierbare Vorrichtung nach einem der vorhergehenden Ansprüche, **dadurch gekennzeichnet, daß** das die Empfangsspule (106) umgebende Polymer (104) und/oder das die flexible Verbindung bildende Polymer und/oder das den Leiter (109) der Empfangsspule (106) umhüllende Material aus der aus Siliconen, Polyurethanen, Polyimiden, Polymethan, Parylen und Polytetrafluorethylen bestehenden Gruppe ausgewählt ist.

17. Implantierbare Vorrichtung nach einem der vorhergehenden Ansprüche, **dadurch gekennzeichnet, daß** die Empfangsspule (106) als kernlose Spule (Luftspule) ausgebildet ist.

## Claims

1. Implantable device with a charging current feed arrangement (78) which has at least one receiving coil (106) or which consists of one such coil, into which via a charging device (59) located outside the body energy can be transcutaneously fed electromagnetically, with an electrochemical battery (90) which can be repeatedly recharged via the charging current feed arrangement, and with a main module (56, 130) supplied by the battery with electric power, the components of the implantable device with the exception of the receiving coil being accommodated in a housing arrangement (72, 72', 72", 128, 132) of biocompatible metal and the receiving coil being arranged outside the housing arrangement and being electrically coupled to the remaining part of the implantable device (54), **characterized in that** the receiving coil (106) is wound from at least one metallic conductor (109) jacketed with an electrically insulating material (111), is surrounded by a biocompatible polymer (104), and is also mechanically connected to at least one part of the housing arrangement (72, 72', 72", 128, 132) so as to form a structural unit with said at least one part.

2. Implantable device as claimed in claim 1, **characterized in that** the metallic housing arrangement (72, 72', 72", 128, 132) is made hermetically tight.

3. Implantable device as claimed in claim 1 or 2, **characterized in that** the receiving coil (106) is fixed on the outside of a metal housing (72, 72', 72", 132) which holds at least the main module (56, 130).

4. Implantable device as claimed in claim 1 or 2, **characterized in that** the receiving coil (106) is fixed on the outside of a metallic outer or protective housing (128) which holds at least the battery (90).

5. Implantable device as claimed in claim 3 or 4, **characterized in that** the receiving coil (106) is fixed on the respective housing (72, 72', 72", 128, 132) via the polymer (104) which surrounds it.

6. Implantable device as claimed in any one of claims 3 to 5, **characterized in that** the receiving coil (106) is placed on at least one part of one broad side (95) of the metal housing (72", 132) of the main module (56, 130) or the metallic outer or protective housing (128) of the battery module (126).

7. Implantable device as claimed in claim 6, **characterized in that** the coil axis (112) of the receiving coil (106) is at least roughly perpendicular to the one broad side (95) of the metal housing (72", 132) of the main module (56, 130) or of the metallic outer or protective housing (128) of the battery module (126).

8. Implantable device as claimed in any one of claims 3 to 5, **characterized in that** the receiving coil (106) is located laterally bordering at least one part of the narrow side (79) of the metal housing (72, 72', 132) of the main module (56, 130) or the metallic outer or protective housing (128) of the battery module (126).

9. Implantable device as claimed in claim 8, **characterized in that** the coil axis (112) of the receiving coil (106) is located at least roughly parallel to the one narrow side (79) of the metal housing (72, 72', 132) of the main module (56, 130) or the metallic outer or protective housing (128) of the battery module (126).

10. Implantable device as claimed in claim 8 or 9, **characterized in that** the receiving coil (106) is kinked by an angle in the range from 5° to 25° relative to the metal housing (72, 72', 132) mechanically connected thereto.

11. Implantable device as claimed in any one of claims 8 to 10, **characterized in that** the receiving coil (106) is fixed flexibly on the metal housing (72, 72', 132) of the main module (56, 130) or on the metallic outer or protective housing (128) of the battery module (126).

12. Implantable device as claimed in claim 11, **characterized in that** the flexible connection between the receiving coil (106) and the respective housing (72, 72', 72",128, 132) consists of biocompatible polymer.

13. Implantable device as claimed in any one of the preceding claims, **characterized in that** the conductor (109) of the receiving coil (106) consists of biocompatible metal.

14. Implantable device as claimed in any one of the preceding claims, **characterized in that** the conductor (109) of the receiving coil (106) is made from a metal which is chosen from the group consisting of gold, platinum, niobium, tantalum, iridium and their alloys.

15. Implantable device as claimed in claim 14, **characterized in that** the conductor (109) of the receiving coil (106) is made of pure gold.

16. Implantable device as claimed in any one of the preceding claims, **characterized in that** the polymer (104) which surrounds the receiving coil (106) and/or the polymer which forms the flexible connection and/or the material which jackets the conductor (109) of the receiving coil (106) is chosen from the group consisting of silicones, polyurethanes, polyimides, polymethane, parylene, and polytetrafluorethylene.

17. Implantable device as claimed in any one of the preceding claims, **characterized in that** the receiving coil (106) is made as a coreless coil (air-core inductor).

## Revendications

1. Dispositif implantable comprenant un dispositif d'injection de courant de charge (78) présentant au moins une bobine de réception (106) ou constitué d'une telle bobine, dans laquelle de l'énergie peut être injectée de façon transcutanée et électromagnétique au moyen d'un dispositif de chargement (59) disposé à l'extérieur du corps, une batterie (90) électrochimique, pouvant être rechargée plusieurs fois au moyen du dispositif d'injection de courant de charge, et un module principal (56, 130) alimenté par la batterie en énergie électrique, les composants du dispositif implantable, à l'exception de la bobine de réception, étant logés dans un dispositif de boîtier (72, 72', 72", 128, 132) à base de métal biocompatible et la bobine de réception étant disposée à l'extérieur du dispositif de boîtier et étant couplée électriquement à la partie restante du dispositif (54) implantable, **caractérisé en ce que** la bobine de réception (106) est enroulée à partir d'au moins un conducteur (109) métallique enveloppé avec un matériau (111) électro-isolant, est entourée par un polymère (104) biocompatible et est reliée en supplément mécaniquement avec au moins une partie du dispositif de boîtier (72, 72', 72'', 128, 132) et forme ainsi une unité de construction avec la au moins une partie.

2. Dispositif implantable selon la revendication 1, **caractérisé en ce que** le dispositif de boîtier (72, 72', 72", 128, 132) métallique est conçu de façon hermétiquement étanche.

3. Dispositif implantable selon la revendication 1 ou 2, **caractérisé en ce que** la bobine de réception (106) est fixée sur un côté extérieur d'un boîtier métallique (72, 72', 72'', 132) recevant au moins le module principal (56, 130).

4. Dispositif implantable selon la revendication 1 ou 2, **caractérisé en ce que** la bobine de réception (106) est fixée sur un côté extérieur d'un boîtier extérieur ou de protection (128) métallique recevant au moins la batterie (90).

5. Dispositif implantable selon la revendication 3 ou 4, **caractérisé en ce que** la bobine de réception (106) est fixée sur le boîtier (72, 72', 72'', 128, 132) concerné au moyen du polymère (104) entourant la bobine.

6. Dispositif implantable selon l'une quelconque des revendications 3 à 5, **caractérisé en ce que** la bobine de réception (106) est posée sur au moins une partie d'un côté large (95) du boîtier métallique (72", 132) du module principal (56, 130) respectivement du boîtier extérieur ou de protection (128) métallique du module de batterie (126).

7. Dispositif implantable selon la revendication 6, **caractérisé en ce que** l'axe de bobine (112) de la bobine de réception (106) est disposé au moins approximativement perpendiculairement à un côté large (95) du boîtier métallique (72'', 132) du module principal (56, 130) respectivement du boîtier extérieur ou de protection (128) métallique du module de batterie (126).

8. Dispositif implantable selon l'une quelconque des revendications 3 à 5, **caractérisé en ce que** la bobine de réception (106) est disposée latéralement de façon contiguë à au moins une partie d'un côté étroit (79) du boîtier métallique (72, 72', 132) du module principal (56, 130) respectivement du boîtier extérieur ou de protection (128) métallique du module de batterie (126).

9. Dispositif implantable selon la revendication 8, **caractérisé en ce que** l'axe de bobine (112) de la bobine de réception (106) est disposé au moins approximativement parallèlement à un côté étroit (79) du boîtier métallique (72, 72', 132) du module principal (56, 130) respectivement du boîtier extérieur ou de protection (128) métallique du module de batterie (126).

10. Dispositif implantable selon la revendication 8 ou 9, **caractérisé en ce que** la bobine de réception (106) est pliée d'un angle allant de 5° à 25° par rapport au boîtier métallique (72, 72', 132) relié mécaniquement à la bobine.

11. Dispositif implantable selon l'une quelconque des revendications 8 à 10, **caractérisé en ce que** la bobine de réception (106) est fixée de façon souple sur le boîtier métallique (72, 72', 132) du module principal (56, 130) respectivement sur le boîtier extérieur ou de protection (128) métallique du module de batterie (126).

12. Dispositif implantable selon la revendication 11, **caractérisé en ce que** la liaison flexible entre la bobine de réception (106) et le boîtier (72, 72', 72", 128, 132) concerné est à base de polymère biocompatible.

13. Dispositif implantable selon l'une quelconque des revendications précédentes, **caractérisé en ce que** le conducteur (109) de la bobine de réception (106) est à base de métal biocompatible.

14. Dispositif implantable selon l'une quelconque des revendications précédentes, **caractérisé en ce que** le conducteur (109) et la bobine de réception (106) est fabriqué à base d'un métal qui est choisi dans le groupe constitué d'or, de platine, de niobium, de tantale, d'iridium et de leurs alliages.

15. Dispositif implantable selon la revendication 14, **caractérisé en ce que** le conducteur (109) de la bobine de réception (106) est fabriqué à base d'or pur.

16. Dispositif implantable selon l'une quelconque des revendications précédentes, **caractérisé en ce que** le polymère (104) entourant la bobine de réception (106) et/ou le polymère formant la liaison flexible et/ou le matériau enveloppant le conducteur (109) de la bobine de réception (106) sont choisis dans le groupe constitué de silicone, de polyuréthannes, de polyimides, de polyméthane, de parylène et de polytétrafluoréthylène.

17. Dispositif implantable selon l'une quelconque des revendications précédentes, **caractérisé en ce que** la bobine de réception (106) est conçue comme bobine sans noyau (bobine d'air).
